# EUROPÄISCHE PATENTANMELDUNG

(11) **EP 2 083 069 A1**
(43) Veröffentlichungstag der Anmeldung: **29.07.2009**
(21) Anmeldenummer: 08150595.0
(22) Anmeldetag: 24.01.2008
(51) Int. Cl.: C12N 1/06, C12M 1/33, C12M 3/08

(54) **Verfahren und Vorrichtung für das Aufschließen von biologischen Zellen**

(71) Anmelder: Qiagen GmbH, 40724 Hilden (DE)
(72) Erfinder: Rothmann, Dr. Thomas, 40724, Hilden (DE); Himmelreich, Dr. Ralf, 40724, Hilden (DE)
(74) Vertreter: Gille Hrabal Struck Neidlein Prop Roos

(57) **Zusammenfassung**

Die Erfindung betrifft ein Verfahren und eine Vorrichtung für das Aufschließen von biologischen Zellen. Unter Aufschließen von Zellen wird das Aufbrechen von Zellen verstanden, so dass Erbinformationen entnommen werden können. Mit der vorliegenden Erfindung sollen insbesondere Nukleinsäuren aus Zellen entnommen werden können. Hierzu gehören RNA und DNA.

Erfindungsgemäß werden Zellen in einem Gefäß aufgeschlossen, welches an eine Leitung angeschlossen und so Teil einer Gesamtvorrichtung sein kann. Über die Leitung kann Flüssigkeit beispielsweise aus dem Behälter abfließen oder in den Behälter gepumpt werden. Im Gefäß befinden sich die üblichen Mittel für die Durchführung eines mechanischen Ausschlusses, also insbesondere Kügelchen sowie eine Pufferlösung. Im Unterschied zum Stand der Technik wird allerdings das Gefäß nicht gerüttelt, also Vibrationen ausgesetzt. Statt dessen wird mit Hilfe eines Magnetrührers gerührt. Starke Vibrationen werden so vermieden. Ein Gefäß zur Durchführung eines Aufschlusses von Zellen kann daher in eine Gesamtvorrichtung problemlos integriert werden.

## Beschreibung

Die Erfindung betrifft ein Verfahren und eine Vorrichtung für das Aufschließen von biologischen Zellen. Unter Aufschließen von Zellen wird das Aufbrechen von Zellen verstanden, so dass Erbinformationen entnommen werden können. Mit der vorliegenden Erfindung sollen insbesondere Nukleinsäuren aus Zellen entnommen werden können. Hierzu gehören RNA und DNA. Desweiteren werden mit der vorliegenden Erfindung Proteine aus dem Zellinneren freigesetzt.

Zellen sind unterschiedlich schwierig aufzuschließen. Kultivierte Zellkulturen können in der Regel einfach mit vergleichsweise die Erbinformation schonenden Mitteln aufgeschlossen werden. Sporen sind dagegen vergleichsweise schwierig aufzuschließen. Die für das Ausschließen von solchen Zellen benötigten Mittel brechen nicht nur die Zellen auf, sondern fragmentieren auch umfangreich Erbinformationen,

Ein Beispiel für einfach aufzuschließende Zellen sind weiße Blutkörperchen, Diese können vergleichsweise schonend aufgeschlossen werden, beispielsweise durch das Enzym Proteinase K in Anwesenheit eines Detergenz (z.B Natrium Dodecylsulfat oder Triton X-100).
Bei jedem Zellaufschluss werden Nukleinsäuren geschert und durch Doppelstrangbrüche im Erbmaterial fragmentiert.
Allerdings erfolgt die Fragmentierung der Erbinformation nicht derart umfangreich, dass dadurch die gewünschten Analysen gefährdet wären.

Je größer das Ausmaß an Fragmentierung der Nukleinsäuren ist, umso problematischer ist es, die gewünschten Analysen durchzuführen. Es ist also ein Ziel bei dem Aufschluss von Zellen, Doppelstrangbrüche auf ein verträgliches Maß zu reduzieren.

Sollen schwer aufzuschließende Zellen aufgeschlossen werden, so geschieht dies beispielsweise durch Zufuhr von Hitze. Dann werden schwer aufzuschfießende Zellen beispielsweise 10 Minuten lang bei 95°C behandelt. Typischerweise werden viele Bakterien mit Hilfe von Hitze aufgeschlossen.

Ein weiteres Verfahren ist der Aufschluss mittels Ultraschall, welches im Fall von schwierig aufzuschließenden Zellen angewendet wird. Ultraschall fragmentiert Erbinformationen allerdings besonders umfangreich. Daher wird Ultraschall üblicherweise nur dann eingesetzt, wenn andere Verfahren versagen. Mit Hilfe von Ultraschall werden beispielsweise Sporen aufgeschlossen, die besonders widerstandsfähig sind.

Ein drittes Verfahren für das Aufschließen von schwierig aufzuschließenden Zellen stellt das Mahlen mittels Glaskugeln dar. Mit Hilfe von Glaskugeln werden Bakterien und Pilze aufgeschlossen. Der Aufschluss mittels Glaskugeln (auch als "Bead Beating" in Fachkreisen bekannt) verläuft vergleichsweise schonend im Vergleich zu Ultraschall und Hitze. In sämtlichen drei Fällen werden jedoch die Erbinformationen unterschiedlich stark fragmentiert.

Soll mit Glaskugeln aufgeschlossen werden, so wird in der Regel eine Rütteloder Schüttelapparatur so zum Beispiel ein sogenannter Vortex^{®}-Rüttler verwendet. Ein oder mehrere aus Kunststoff bestehende Röhrchen werden mit einer wässrigen Lösung, die einen Lysepuffer umfasst, dem Zellmaterial und Glaskügelchen gefüllt, verschlossen und in die Rüttel- oder Schüttelapparatur gestellt und mittels der Apparatur beispielsweise für fünf Minuten geschüttelt. Ein solches Verfahren wird auch als "Vortexen mit Glasbeads" oder als "Bead Beating" bezeichnet.

Ist der Zellaufschluss abgeschlossen, so werden das oder die Röhrchen entnommen, die Röhrchen bei Bedarf zentrifugiert, um Glaspartikel von der Flüssigkeit zu trennen, und der flüssige Inhalt wird in ein weiteres Röhrchen gegeben. Hier wird das aufgeschlossene Zellmaterial mit weiteren Pufferlösungen behandelt, um die Nukleinsäure zu prozessieren.

Ein Aufschließen mittels Glaskügelchen wird beispielsweise in der Druckschrift DE 10 2004 032 888 B4 beschrieben.

Zwar handelt es sich bei diesem Verfahren um eine bewährte und zuverlässig funktionierende Methode. Diese kann aber kaum automatisiert werden und vor allem nicht, wenn eine Automatisierung nur wenig Platz beanspruchen soll oder darf. Problematisch sind vor allem die starken Vibrationen, die Schäden verursachen können, wenn als Lysegefäße dienende Röhrchen Teil einer Gesamtvorrichtung sind.

Aufgabe der Erfindung ist die Schaffung eines Verfahrens und einer Vorrichtung für das effiziente Aufschließen von Zellen.

Zur Lösung der Aufgabe werden Zellen in einem Gefäß aufgeschlossen, welches an eine Leitung angeschlossen und so Teil einer Gesamtvorrichtung sein kann. Über die Leitung kann Flüssigkeit beispielsweise aus dem Gefäß abfließen oder in das Gefäß gepumpt werden. Im Gefäß befinden sich die bekannten Mittel für die Durchführung eines mechanischen Ausschlusses, also insbesondere Kügelchen sowie eine Pufferlösung. Als Alternative zu Glaskügelchen können auch aus Zirkonium bestehende Kügelchen oder SiliziumCarbid-Partikel in geeigneter Größe einsetzt werden, Im Unterschied zum Stand der Technik wird allerdings das Gefäß nicht gerüttelt, also Vibrationen ausgesetzt. Statt dessen wird insbesondere mit Hilfe eines Magnetrührers gerührt. Starke Vibrationen werden so vermieden. Ein Gefäß zur Durchführung eines Aufschlusses von Zellen kann daher in eine Gesamtvorrichtung bzw. Gesamtsystem problemlos integriert werden.

Es ist problematisch, ein Gefäß, welches beispielsweise mit einer Leitung verbunden ist, wie aus dem Stand der Technik bekannt zu rütteln, also Vibrationen auszusetzen. Dies gilt vor allem dann, wenn eine Miniaturisierung angestrebt wird. Allerdings war die Fachwelt der Meinung, Vibrationsbewegungen, die ständig wechselnde Bewegungsrichtungen der Glaskügelchen erzeugen, seien erforderlich, um effizient selbst Zellen von Bakterien sowie Pilzen aufschließen zu können.

Mit Hilfe eines Magnetrührers oder eines anderen Rührwerkzeugs lassen sich Vibrationsbewegungen der Glaskügelchen, wie sie mit Hilfe von Rüttelapparaten erzeugt werden können, nicht annähernd nachbilden. Ein Magnetrührer dient nach dem Stand der Technik daher dem Mischen von Inhalten in einem Gefäß, nicht aber als Mittel, um einen mechanischen Aufschluss von Zellen herbeizuführen. Daher ist aus dem Stand der Technik insbesondere nicht bekannt, mit Hilfe eines Magnetrührers oder gleich wirkenden Rührwerkzeugen Partikel oder Kügelchen zu bewegen, um so Zellen mechanisch aufzuschließen.

Überraschend hat sich gezeigt, dass der mit einem Rührstab eines Magnetrührers bewirkte mechanische Aufschluss genauso effizient wie der aus dem Stand der Technik bekannte mechanische Aufschluss mit einer Rüttelapparatur ist, Es wurde untersucht, ob eine häufige Änderung der Drehrichtung des Magnetrührers für ein effizientes Aufschließen erforderlich ist, um so eine Vibrationsbewegung nachzuahmen. Es stellte sich unerwartet heraus, dass wechselnde Drehrichtungen des Magnetrührers die Effizienz nur wenig beeinflussen. Es genügt also bereits, nur eine Drehrichtung des Magnetrührers vorzusehen, um effizient aufschließen zu können.

Durch den Einsatz des Magnetrührers wird erreicht, dass das Gefäß nicht gerüttelt wird. Es ist daher nun unproblematisch, das Gefäß in eine mechanisch empfindliche Gesamtvorrichtung zu integrieren. Andernfalls entstehen durch das Rütteln sehr leicht Beschädigungen, so zum Beispiel undichte Übergänge zwischen Leitungen, wie Versuche gezeigt haben. Außerdem können empfindliche Teile einer Gesamtvorrichtung für eine automatische oder halbautomatische Prozessierung leicht durch Rütteln beschädigt werden.

Der Magnetrührer umfasst in einer Ausführungsform einen magnetischen Rührstab. In einer vorteilhaften Ausgestaltung der Erfindung ist die Länge des Rührstabs so gewählt, dass dieser nicht horizontal auf dem Grund des Gefäßes liegen kann. Der Rührstab liegt dann in der Regel im Ruhezustand mit einem Ende auf dem Grund des Gefäßes - welcher ein Gefäßboden, ein Filter oder eine Fritte sein kann - und grenzt mit dem anderen Ende an eine Gefäßwand an. Die Länge des Rührstabs ist also je nach Ausführungsform länger als der Durchmesser des Bodens des Gefäßes oder aber des Durchmessers einer horizontal im bestimmungsgemäß aufgestellten Gefäß gelagerten Fritte. Es hat sich herausgestellt, dass bei dieser Ausgestaltung die Effektivität des Aufschlusses regelmäßig deutlich verbessert werden kann.

Ist das Flüssigkeitsvolumen allerdings sehr klein, so kann es auch genügen, einen Rührstab eines Magnetrührers horizontal liegend einzusetzen, ohne merkliche Einschränkungen in Bezug auf die Effektivität des Verfahrens hinnehmen zu müssen.

In einer Ausgestaltung der Erfindung weist das Gefäß beispielsweise am Boden einen Filter oder eine Fritte auf. Der Filter bzw. die Fritte dient dazu, Partikel bzw. Kügelchen herauszufiltern, wenn der flüssige Inhalt beispielsweise über eine Leitung abgesaugt wird. Es entfällt so das aus dem Stand der Technik bekannte Zentrifugieren, um Glaskügelchen von der Flüssigkeit zu trennen. Außerdem wird so verhindert, dass eine Leitung durch die Partikel bzw. Kügelchen verstopft wird.

Der Durchmesser der vorzugsweise aus Glas bestehenden Partikel bzw. Kügelchen liegt typischerweise bei etwa 600 µm. Der Durchmesser der Poren des Filters bzw. der Fritte beträgt daher bevorzugt bis zu 100 µm und zwar insbesondere 50 bis 100 µm, um die Flüssigkeit nebst darin befindlichem Zellmaterial zuverlässig von den Partikeln zu trennen.

Als Filter eignet sich insbesondere eine aus Polypropylen bestehende Fritte.

Um besonders effektiv aufschließen zu können, sollte der Flüssigkeitsstand im Gefäß niedrig sein. Dies gelingt zwar mit einer großen Grundfläche des Gefäßes. Dann aber ist es schwierig, den flüssigen Inhalt möglichst vollständig beispielsweise über eine am Boden angebrachte Leitung abzusaugen. Daher hat sich eine konische Form des Gefäßes als vorteilhaft herausgestellt, wobei der Durchmesser des Gefäßes nach oben zunimmt. Flach ansteigende oder ebene Flächen im Gefäß werden so vorteilhaft vermieden bzw. minimiert. Die Grundfläche des Gefäßes kann klein sein, ohne einen hohen Flüssigkeitsstand im Gefäß hinnehmen zu müssen.

Das Gefäß wird vorzugsweise wie vom Stand der Technik her bekannt während des Aufschließens durch einen entsprechenden Deckel verschlossen.

Grundsätzlich ist es möglich, anstelle des Magnetrührers auch ein anderes Mischwerkzeug für das Rühren vorzusehen. Ein Magnetrührer hat sich allerdings als besonders geeignet herausgestellt, Denn dann muss keine Welle beispielsweise durch einen Deckel hindurch in das Gefäß reichen, was unerwünschte Vibrationen zur Folge haben kann, die durch Einsatz eines Magnetrührers vermieden werden. Desweiteren arbeitet ein Magnetrührer in Sinne der Aktorik berührungsfrei. Das Gefäß ist während der Vorgangs verschlossen wodurch auch kein Probenmaterial entweichen kann. Dies ist insbesondere vorteilhaft, wenn es sich um potentiell infektiöse Probenmaterial handelt.

Ein für das Rühren eingesetztes Rührelement kann verschiedene Rührbewegungen ausführen. Inbesondere werden kreisförmige Rührvorgänge durchgeführt. Vorzugsweise wird ein im Gefäß befindliches Rührelement berührungsfrei angetrieben. Das Rührelement kann verschiedene Längen bezogen auf den Durchmesser des Gefäßes haben. Das Rührelement kann verschieden Geometrien haben kann und weist vorzugsweise die Geometrie eines Stabes auf, Das Gefäß und insbesondere die Gefäßinnenwand kann unterschiedlich ausgestaltet sein. Vorzugsweise weist es eine glatte Innenwand auf und es handelt sich um ein konisches Gefäß. Eine im Gefäß befindliche Lösung kann zusätzlich Enzyme für den Zellaufschluss enthalten.

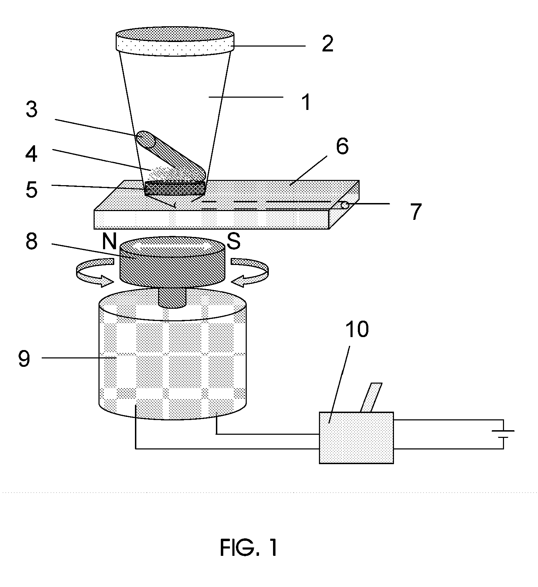

Figur 1 zeigt schematisch den Aufbau der Vorrichtung für eine automatisierte Prozessierung von biologischen Proben. Ein konisches Gefäß 1 ist mit einem Deckel 2 verschlossen, um so einen unerwünschten Austritt von Flüssigkeit und anderem Material aus dem Gefäß - nachfolgend auch Lysekammer genannt - zu verhindern, In dem Gefäß 1 (unterer Durchmesser 0,7 cm; oberer Durchmesser 2,0 cm; Innenhöhe 2,5 cm; Volumen ca. 3,2 ml) befindet sich ein magnetischer Rührstab 3, mit dem während des Aufschließens von Zellen gerührt wird. Des weiteren enthält das Gefäß 1 Glaskügelchen 4, die auf einer aus Polypropylen bestehenden Fritte 5 aufliegen. Der Durchmesser der Fritte beträgt 7 mm. Die Fritte 5 ist 1,5 mm dick und ist mit durchgehenden Poren mit einem Durchmesser von 50 bis 100 µm versehen. Da der magnetische Stab 3 länger als der Durchmesser der Fritte 5 ist, kann dieser nicht horizontal auf der Fritte aufliegen, die den Gefäßgrund bildet. Das Gefäß 1 befindet sich auf einem Substrat 6, welches unterhalb der Fritte mit einer trichterförmigen Mulde versehen ist. Das untere Ende der trichterförmigen Mulde mündet in einen Kanal 7 ein. Unterhalb des Substrates 6 sowie des Gefäßes 1 ist ein Magnet 8 horizontal ausgerichtet an der Achse eines Elektromotors befestigt. Die Drehrichtung des Elektromotors kann mit einem Umpolschalter 10 verändert werden.

Die in Figur 1 gezeigte Vorrichtung ist Teil eines mikrofluidischen Systems, weiches weitere Einrichtungen umfasst oder an Einrichtungen angeschlossen ist, um automatisiert oder halbautomatisch prozessieren zu können, Hierzu gehört insbesondere eine Pumpe, mit der eine Lysepufferlösung abgepumpt werden kann. Auch umfasst das System in einer Ausführungsform einen Behälter mit einer darin enthaltenen Lysepufferlösung, um die Pufferlösung in das Gefäß 1 pumpen zu können.

Die Innendurchmesser der Kanäle des mikrofluidischen Systems sind nicht größer als 1 mm und zwar insbesondere auch der Innendurchmesser des in Figur 1 gezeigten Kanals 7.

Bevorzugt handelt es sich sowohl bei dem Substrat 6, auf das das Gefäß 1 aufgesetzt ist, sowie bei dem Gefäß 1 um Teile, die durch Spritzguss hergestellt wurden bzw. werden können, um eine preiswerte Fertigung zu ermöglichen. Diese Teile werden geeignet zusammengesetzt, so dass eine Öffnung am Grund des Gefäßes 1 mit einer Öffnung eines Mikrokanals 7 verbunden wird. Anschließend werden die Teile 1 und 6 bevorzugt durch Wärme miteinander verschweißt, so dass eine zuverlässig dichte Verbindung zwischen dem Kanal 7 und dem Gefäß 1 entsteht. Das Material des Gefäßes 1 ist typischerweise Polypropylen. Als Material für das Substrat werden Polycarbonat oder Cyclo-Olefin-Copolymere (COC) bevorzugt. Polycarbonat und COC sind durchsichtig, hart und formstabil. Diese sind damit den Anforderungen gewachsen, die an ein Substrat für automatische Abläufe gestellt werden und zwar vor allem an die Maßhaltigkeit. Der Fachmann kann für den Spritzguss auch alternative Polymere mit den gleichen Anforderungen benutzen. Die Anforderungen an die Maßhaltigkeit des Lysegefäßes 1 sind geringer. Daher genügt Polypropylen als Material für das Gefäß 1.

Das Gefäß 1 wird in der Regel einer maximalen Temperatur von 80°C ausgesetzt. Das Substrat 6 kann dagegen im Rahmen einer automatischen Prozessierung Temperaturen von bis zu 100°C ausgesetzt sein. Aus diesem Grund wird als Substratmaterial ein relativ thermostabiles Material bevorzugt.

Der Kanal 7, an den das Gefäß 1 angeschlossen ist, kann über ein entsprechendes Ventil geöffnet und geschlossen werden. Während des Aufschließens von Zellen ist der Kanal 7 verschlossen. In das Gefäß werden für das Aufschließen beispielsweise in bekannter Weise die Probe, die die Zellen enthält, eingefüllt sowie ein Lysepuffer und Glaskügelchen.

Zum Nachweis der Effizienz der erfindungsgemäßen Vorrichtung bzw. des erfindungsgemäßen Verfahrens wurden verschiedene Proben nach dem Stand der Technik sowie mit Hilfe der Erfindung prozessiert, wie nachfolgend näher beschrieben wird.

Nachfolgend werden mit der Erfindung erreichte Ergebnisse auch unter Einbeziehung von Vergleichsversuchen verdeutlicht.

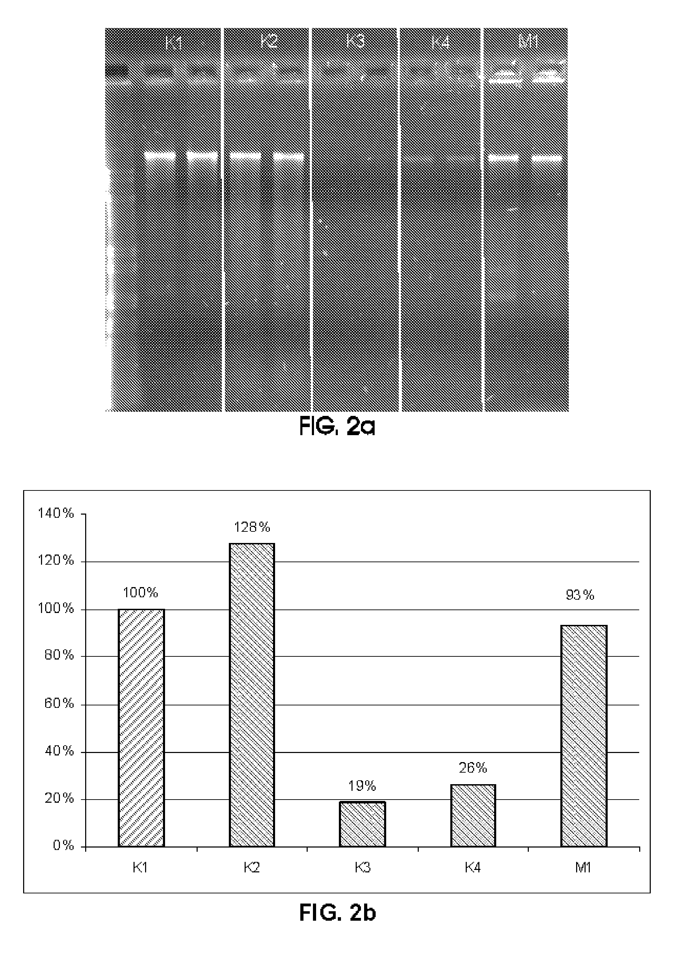

Eine erste Versuchsreihe bezieht sich auf die Nukleinsäure-Präparation von Corynebacterium glutamicum, Die erzielten Ergebnisse werden in den Figuren 2a und 2b dargestellt.

Die Präparationen erfolgten zum einen gemäß dem folgenden, dem Stand der Technik entsprechenden Protokoll zur Nukleinsäure-Präparation aus Flüssigkulturen von Mikroorganismen.
1. 250µl Bakterien oder Hefe Flüssigkultur in 1,5 ml Mikrozentrifugationsröhrchen aliquotieren
2. Zentrifugation für 10 min bei 5000 x g und Überstand verwerfen
3. Zellsediment in 600µl Lyse-Puffer resuspendieren [Bakterien Lysepuffer und Fungi Lysepuffer unterscheiden; bei Hefe optional 20µl Zymolase benutzen]
4. Zugabe von 200mg GlassBeads (Glaskügelchen mit einem Durchmesser von 600µm) [Sigma-Aldrich Art. Nr. G8772-500G]
5. 5 min bei maximaler Leistung auf einem Vortex-Mischer mit Scheibenaufsatz für 1,5 ml Mikrozentrifugotionsröhrchen mischen.
6. Zugabe von 600µl Puffer AL [QIAGEN; QIAamp DNA Micro Kit]
7. Zugabe von 20µl Proteinase K [QIAGEN; QIAamp DNA Micro Kit]
8. Mischen und Inkubation für 10min bei 56°C
9. Kurze Zentrifugation für 20 sec.
10. Transfer des gesamten Überstands in ein neues 1,5 ml Mikrozentrifugationsröhrchen
11. Zugabe von 600µl Ethanol
12. Gemisch auf QIAcmp MinElute Spin Column geben (mehrmals)
13. *Bindung: 1 min bei 6000 x g zentrifugieren
14. *Waschschritte: 500µl Puffer AW1, 1 min bei 6000 x g ; 500µl Puffer AW2, 3min bei max. g-Zahl
15. *"Dry Spin für 1 min maximaler g-Zahl
16. *Zur Elution der Nukleinsäuren mit 80µl Puffer TE eluieren, 1 min bei 6000 x g
*) Schritte gemäß dem "QIAamp DNA Micro Handbook", welches u. a. im Internet über die Adresse http://wwwl.qiogen.com/HB/QIAampDNAMicroKit_EN erhalten werden kann,

### Lyse Puffer:

Lyse Puffer für Bakterien z.B. Corynebacterium glutamicum 1,2% Triton mit 20 mg/ml Lysozym
Zugabe von 10µl Reagent DX (Silicon-Antischaum Emulsion; Wacker Chemie AG) auf 6ml Lysepuffer
Lyse Puffer für Fungi z.B, Saccharomyces cerevisiae 1 M Sorbitol; 100mM EDTA
Optional zusätzlich 20µl Zymolose(2,5mg/ml)/Probe

Nukleinsäure wurde außerdem gemäß der Erfindung nach dem folgenden Protokoll prozessiert (Protokoll im Sinne der Erfindung):
1. 250µl Bakterien oder Hefe Flüssigkultur in 1,5 ml Mikrozentrifugationsröhrchen aliquotieren
2. Zentrifugation für 10 min bei 5000 x g und Überstand verwerfen
3. Zellsediment in 600µl Lyse-Puffer resuspendieren und in Lysekammer (Gefäß 1 wie in Figur 1 gezeigt) transferieren [Bakterien Lysepuffer und Fungi Lysepuffer unterscheiden; bei Hefe optional 20µl Zymolase benutzen]
4. Zugabe von 200mg GlassBeads, Durchmesser 600µm [Sigma-Aldrich Art. Nr. G8772-500G] in Lysekammer
5. 10min Magnetrühren (zentrisch) bei größtmöglicher Umdrehungszahl, ohne dass es zur Verspritzung des Lysates kommt; Rührrichtung durch Umpolung der Elektromotors alle 20 sec ändern
6. Zugabe von 600µl Puffer AL [QIAGEN; QIAomp DNA Micro Kit] Transfer mit aufgeschnittener Spitze in 1,5 ml Mikrozentrifugationsröhrchen
7. Zugabe von 20µl Proteinase K [QIAGEN; QIAamp DNA Micro Kit]
8. Mischen und Inkubation für 10min bei 56°C
9. Kurze Zentrifugation für 20 sec.
10. Transfer des gesamten Überstands in ein neues 1,5 ml Mikrozentrifugationsröhrchen
11. Zugabe von 600µl Ethanol
12. Gemisch auf QIAamp MinElute Spin Column geben (mehrmals)
13. *Bindung: 1 min bei 6000 x g zentrifugieren
14. *Waschschritte: 500µl Puffer AW1, 1 min bei 6000 x g ; 500µl Puffer AW2, 3min max. g-Zahl
15. *"Dry Spin für 1 min maximaler g-Zahl
16. *Zur Elution der Nukleinsäuren mit 80µl Puffer TE eluieren, 1min bei 6000 x g

Nach der Präparation gemäß den vorbeschriebenen Protokollen wurden die Nukleinsäuren auf einem Agarosegel elektrophoretisch aufgetrennt. Nach der Auftrennung wurden die Nukleinsjurefragmente in einem Ethidiumbromid-haltiges Wasserbad angefärbt, auf einer UV-Leuchtplatte zum Leuchten gebracht und durch ein Fotodokumentationssystem abgelichtet. Die genomische DNA ist als helle Bande im oberen Drittel der Figur 2a zu sehen.

Fig. 2a bezieht sich auf die Nukleinsäure-Präparation von Corynebacterium glutamicum, 0,7% Agarosegel (Auftrag 15µl Eluat). Dabei wird eine Lösung von 0.7% w/v Agarose bis zum Schmelzen der Agarosepartikel erhitzt, und nach der Homogenisierung lässt man sie in einer Gelform erstarren. Das Gel enthält Taschen, in die die Nukleinsäure-haltige Lösung pipettiert und elektrophoretisch auftrennt wurde, In diesem Fall wurde 15 µl des Eluates nach Nukleinsäure Präp "aufgetragen", wobei
K1: Referenzprotokoll mit Vortex-Mischer ohne Lysozym im Lysepuffer
K2: Referenzprotokoll mit Vortex-Mischer mit Lysozym im Lysepuffer
K3: Referenzprotokoll ohne Vortex-Mischen und ohne Lysozym im Lysepuffer
K4: Referenzprotokoll ohne Vortex-Mischen aber mit Lysozym im Lysepuffer
M1: Protokoll im Sinne der Erfindung ohne Lysozym im Lysepuffer,

Die Stärke, mit anderen Worten die Leuchtintensität (Anzahl und Fläche weisser Pixel) einer Bande in der Figur 1A ist ein Maß für die Qualität des Zellaufschlusses, In erster Linie maßgeblich ist die helle Bande bei der 1/3 zu 2/3 Grenze der Bildvertikalen.

Die Banden K1 und K2 bzw. die Banden, die aus den Versuchen K1 und K2 resultierten, sind hell und breit im Vergleich zu den Banden K3 und K4, also den Banden, die im Rahmen der Versuche K3 und K4 erhalten wurden. Die Banden K3 und K4 wurden im Unterschied zu den Banden K1 und K2 ohne Vortex-Mischen erhalten. Dies verdeutlicht, dass ein mechanischer Aufschluss unverzichtbar ist, um mit brauchbaren Ergebnissen aufschließen zu können. Die Banden K1 und K2 entsprechen der Bande M1, die durch das erfindungsgemäße Verfahren mit der in Figur 1 gezeigten Vorrichtung erhalten wurde. Hieraus folgt, dass der mechanische Aufschluss gemäß der vorliegenden Erfindung in qualitativer Hinsicht keinen Unterschied zu dem mechanischen Aufschluss nach dem Stand der Technik erkennen lässt.

Um weiter zu verdeutlichen, dass der mechanische Aufschluss in qualitativer Hinsicht entscheidend ist, wurden Vergleichsversuche mit und ohne Lysozym durchgeführt. Lysozym ist eine Hydrolase, die die Zellwand von Bakterien angreift. Der Vergleich der Bande K3 (Lysozym wurde nicht eingesetzt) mit K4 (Lysozym wurde eingesetzt) zeigt einen erkennbaren Einfluss im Fall eines ansonsten wenig brauchbaren Aufschlusses, da die Bande K4 im Unterschied zur Bande K3 in der Darstellung noch schwach erkennbar ist. Wird aber mechanisch aufgeschlossen und damit in brauchbarer Weise, so ist ein Einfluss in Figur 2a nicht mehr zu erkennen, wie der Vergleich der Bande K1 (Lysozym wurde nicht eingesetzt) mit der Bande K2 (Lysozym wurde eingesetzt) zeigt. Der Einsatz von Lysozym macht sich in qualitativer Hinsicht also nicht bemerkbar.

Oberhalb der Bande M1, also der Bande, die im Rahmen des erfindungsgemäß durchgeführten Versuchs erhalten wurde, ist eine weitere Bande direkt "an der Geltasche" erkennbar. Das bedeutet, dass das Aufschlussverfahren im Vergleich zu dem Vortex-Mischen gemäß Stand der Technik schonender verläuft, also weniger geschert wird. Insoweit ist das erfindungsgemäße Verfahren dem Stand der Technik sogar überlegen.

Fig. 2b bezieht sich ebenfalls auf die genannte Nukleinsäure-Präparation von Corynebacterium glutamicum. Gezeigt werden die gemessenen optischen Dichten (OD) relativ zu der optischen Dichte, die im Rahmen des Versuchs K1 erzielt wurde. Die optische Dichte wurde jeweils mit einer Wellenlänge des Lichts von 260nm ermittelt. Die optische Dichte ist ein Maß für die Effizienz des Zellaufschlusses in quantitativer Hinsicht. Der Vergleich der optischen Dichten im Fall der Versuche K1 und K2 verdeutlicht, dass sich die Verwendung von Lysozym in quantitativer Hinsicht positiv auswirkt, da die optische Dichte des Versuchs K2 mit 128% deutlich höher im Vergleich zu der optischen Dichte des Versuchs K1 ist, bei dem zwar ebenfalls mechanisch nach dem Stand der Technik aufgeschlossen, aber eben im Unterschied zum Versuch K2 kein Lysozym eingesetzt wurde. Der erfindungsgemäß durchgeführte Aufschluss M1, bei dem kein Lysozym eingesetzt wurde, führte zu einer optischen Dichte, die mit 93% an die optische Dichte des Versuchs K1 heranreicht, In quantitativer Hinsicht wurde also ein Ergebnis in gleicher Größenordnung erzielt.

Die Versuche K3 und K4, die ohne mechanischen Aufschluss durchgeführt wurden, führten dagegen zu geringeren optischen Dichten von nur 19% im Fall des fehlenden Lysozyms bzw. 26% im Fall der Verwendung von Lysozym im Vergleich zu Dichte, die mit dem Versuch K1 erzielt wurde. Um also ein quantitativ gutes Ergebnis zu erzielen, ist der mechanische Aufschluss entscheidend. Zwar lässt durch zusätzliche Verwendung von Lysozym eine merkliche Verbesserung auch im Fall des mechanischen Aufschlusses erzielen. Maßgeblich bleibt allerdings auch in quantitativer Hinsicht der mechanische Aufschluss. Wird der Aufschluss erfindungsgemäß durchgeführt, so wird ebenfalls ein sehr gutes Ergebnis erzielt, welches an den mechanischen Aufschluss gemäß Stand der Technik heranreicht.

Mit Hilfe der Erfindung können also problematisch aufzuschließende Bakterien erfolgreich aufgeschlossen werden.

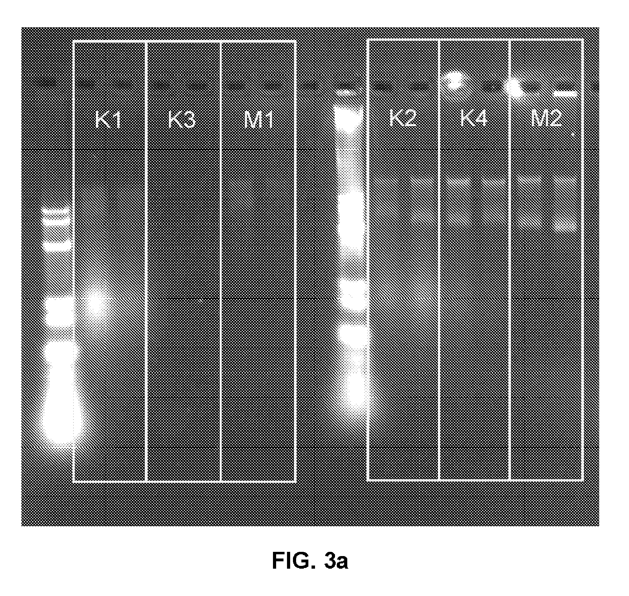

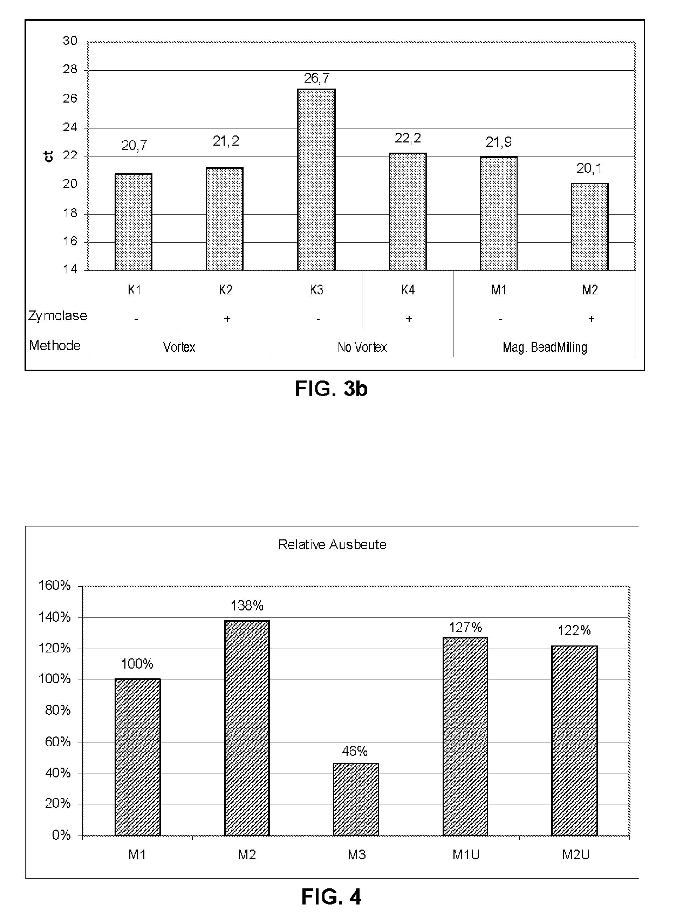

Eine zweite durchgeführte Versuchsreihe bezieht sich auf die Nukleinsäure-Präparation von Saccharomyces cerevisiae (Hefe), 0,7% Agarosegel (Auftrag 15µl Eluat). Die oben genannten Protokolle wurden angewendet. Die erzielten Ergebnisse werden in den Figuren 3a und 3b dargestellt.

Folgende Versuche wurden durchgeführt:
K1: Referenzprotokoll mit Vortex-Mischer ohne Zymolase im Lysepuffer
K2: Referenzprotokoll mit Vortex-Mischer mit Zymolase im Lysepuffer
K3: Referenzprotokoll ohne Vortex-Mischen und ohne Zymolase im Lysepuffer
K4: Referenzprotokoll ohne Vortex-Mischen aber mit Zymolase im Lysepuffer
M1: Protokoll im Sinne der Erfindung ohne Zymolase im Lysepuffer
M2: Protokoll im Sinne der Erfindung mit Zymolase im Lysepuffer.

Figur 3a stellt in Übereinstimmung mit Figur 2a das qualitativ erzielte Ergebnis der Nukleinsäure-Präparation von Saccharomyces cerevisiae, 0,7% Agarosegel (Auftrag 15µl Eluat) dar.

Figur 3b bezieht sich auf die Nukleinsäure-Präparation von Saccharomyces cerevisiae. Dargestellt werden die Ergebnisse der quantiativen Realtime-PCR zum Nachweis genomischer Saccharomyces cerevisiae, Gezeigt wird der sogenannte "Threshold Cycle" (Ct).

Die Polymeraso-Kettenrecktion (englisch Polymerase Chain Reaction, PCR) ist eine Methode, um die Erbsubstanz DNA in vitro zu vervielfältigen, d.h, ohne einen lebenden Organismus wie zum Beispiel das Bakterium Escherichia coli oder die Bäckerhefe Saccharomyces cerevisiae zu benutzen. Die Realtime-PCR (kurz RTD-PCR) ist eine Vervielfültigungsmethode für Nukleinsäuren, die auf dem Prinzip der herkömmlichen Polymerase-Kettenrecktion (PCR) beruht, und zusätzlich die Möglichkeit der Quantifizierung bietet. Die Quantifizierung wird mit Hilfe von Fluoreszenz-Messungen am Ende bzw. während eines PCR-Zykluses (daher der Name "Real Time") durchgeführt und unterscheidet sich somit von anderen quantitativen PCR-Methoden (qPCR), die erst nach Ablauf der PCR quantitativ ausgewertet werden (z. B. Kompetitive PCR), Die Fluoreszenz nimmt proportional mit der Menge der PCR-Produkte zu, was eine Quantifizierung möglich macht. Eine gelelektrophoretische Auftrennung der Fragmente ist nicht nötig, die Daten sind sofort verfügbar und das Kontaminationsrisiko ist gering.

In der ersten Phase der Amplifikation einer PCR ist die Templatemenge begrenzt und die Wahrscheinlichkeit, dass sich Template, Primer und Polymerase treffen, suboptimal, während in der dritten Phase der Amplifikation die Menge der Produkte (DNA, Pyrophosphat, Monophosphatnucleotide) derart ansteigt, dass es zur Hemmung durch diese kommt, häufiger Produktfragmente miteinander hybridisieren, die Substrate langsam verbraucht werden und letztlich die Polymerasen und Nucleotide durch die Hitze langsam zerstört werden. Ein exponentieller und daher quantifizierbarer Anstieg findet sich nur in der Phase dazwischen. Exponentiell bleibt eine PCR bei 12 bis 400 Ausgangskopien für ca, 30 Zyklen, bei 200 bis 3200 für 25 Zyklen und bei anfänglich 3200 bis 51200 für höchstens 20 Zyklen. Um immer am Anfang der exponentiellen Phase messen zu können, wird der CT-Wert (Threshold Cycle = "Schwellenwert-Zyklus") bzw. der Cp-Wert (Crossing Point) verwendet, der den Zyklus beschreibt, an dem die Fluoreszenz erstmalig signifikant über die Hintergrund-Fluoreszenz ansteigt.

Figur 2a verdeutlicht, dass auch im Fall der Hefe, also im Fall eines Pilzes mit Hilfe des erfindungsgemäßen Verfahrens das qualitativ beste Ergebnis erzielt wurde, da im Fall von M2 im Unterschied zu den anderen Versuchen im Bereich der Taschen eine weitere Bande deutlich erkennbar ist. Im Fall der Hefe wirkt sich allerdings die Verwendung des Enzyms Zymolase deutlich auf die Qualität der Versuchsergebnisse aus, wie der Vergleich der Fälle K1 K3, M1 mit den Fällen K2, K4 und M2 zeigt. In den erstgenannten drei Fällen wurde im Unterschied zu den letzten drei Fällen das Enzym nicht eingesetzt. In qualitativer Hinsicht ist der Einfluss aufgrund des mechanischen Mischens vergleichsweise gering, wie insbesondere der Vergleich zwischen K2 und K4 verdeutlicht.

Figur 3b zeigt ein Maß für die Ergebnisse in quantitativer Hinsicht. Je kleiner der Wert ct ist, desto besser ist die quantitative Ausbeute. Bei der PCR ist der exponentielle Zusammenhang der Werte zu bedenken. So ist die Differenz der Werte K3 - K1 = 26, 7 - 20,7 = 6 Ct's. Dies bedeutet, dass die Ausbeute im Fall K3 um den Faktor 2⁶ = 64 geringer ist. Ein Delta-Ct von ca. 0,4 - 0,5 ist nicht signifikant, da ein solcher Wert innerhalb der Streuung liegt. Beträgt der Delta-Ct bei 1, wie bei dem Vergleich M1 mit K1, so liegt die Ausbeute bei 50% (weniger).

In quantitativer Hinsicht wurden die besten Resultate mit dem Versuch M2 erzielt, also einem erfindungsgemäß durchgeführten Verfahren, bei dem Zymolase eingesetzt wurde. Der Vergleich der Fälle K2 mit K4 sowie K1 mit K3 verdeutlicht, dass ein mechanischer Aufschluss einen erheblichen Einfluss auf das quantitative Ergebnis hat.

Diese Versuchsreihe verdeutlicht, dass das erfindungsgemäße Verfahren im Fall eines Hefepilzes dem Stand der Technik jedenfalls dann sowohl in qualitativer als auch in quantitativer Hinsicht überlegen ist, wenn zusätzlich zum mechanischen Einfluss ein für den Aufschluss geeignetes Enzym eingesetzt wird.

Im Rahmen einer dritten Versuchsreihe wurde untersucht, wie sich ein Wechsel der Drehrichtung eines Magnetrührers auf die Versuchsergebnisse auswirkt.

Durch Umpolung des Elektromotors kann die Rührrichtung entweder unidirektional oder bidirektional ausgeführt werden, Das Protokoll wurde gemäß der ersten Versuchsreihe durchgeführt. Untersucht wurde konstantes Rühren sowie bidirektionales Rühren durch Umpolung (während des zehnminütigen Aufschlusses wurde alle 20 Sekunden die Drehrichtung durch Umpolung geändert) und zwar mit und ohne Glasspartikel.

Im Figur 4 sind die quantitativ erzielten ct-Ergebnisse relativ dargestellt, die aus einer durchgeführten Realtime-PCR zum Nachweis genomischer Corynebacterium glutamicum DNA resultierten. Gezeigt wird die relative Ausbeute normiert auf den Wert "M1".

Die Darstellungen in Figur 4 beziehen sich auf folgende Versuche,
M1: Protokoll im Sinne der Erfindung ohne Umpolung der Rührrichtung und ohne Lysozym im Lysepuffer
M2: Protokoll im Sinne der Erfindung ohne Umpolung der Rührrichtung und mit Lysozym im Lysepuffer
M3: Protokoll im Sinne der Erfindung ohne Umpolung der Rührrichtung und ohne Glasspartikel (Glasbeads) und ohne Lysozym im Lysepuffer
M1U: Protokoll im Sinne der Erfindung mit Umpolung der Rührrichtung und ohne Lysozym im Lysepuffer
M2U: Protokoll im Sinne der Erfindung mit Umpolung der Rührrichtung und mit Lysozym im Lysepuffer

Ein Vergleich der Resultate von M1 mit M 1 U bzw. M2 mit M2U zeigt, dass eine Umpolung der Rührrichtung nicht erforderlich ist, um zu guten Ausbeuten zu gelangen. Zwar ergab sich den Messwerten nach zu urteilen im Fall M1/ M2U eine Verbesserung der Ausbeute durch Umpolung. Im Fall M2/M2U war das Ergebnis jedoch umgekehrt.

Weiterhin zeigt der Vergleich M3 zu M1, dass die Anwesenheit von Partikeln sehr wichtig für die Verbesserung der Ausbeute ist. Mit einem Rührwerkzeug alleine ist ein zufriedenstellender Aufschluss also nicht möglich,

Im Rahmen einer vierten Versuchreihe wurde eine Nukleinsäure-Präparation von Mikroorganismen aus Blut untersucht.

Das Vortex-Referenzprotokoll kommt neben der Anwendung zur Isolierung von Nukleinsäuren aus Flüssigkulturen von schwer zu lysierenden Mikroorganismen auch noch bei der Untersuchung von Patientenblut mit Verdacht auf Sepsis zur Anwendung. Aus diesem Grund wurde in diesem Experiment humanes Blut (Blutkonserve) mit Corynebacterium glutamicum versetzt. Die Nukleinsäuren wurden mit verschiedenen Protokollen isoliert und mittels quantitativer PCR nachgewiesen.

Protokoll gemäß dem Stand der Technik:
1. 200µl humanes Blut und 20 µl Corynebacterium glutamicum Übernachtkultur in 1,5 ml Mikrozentrifugationsröhrchen aliquotieren
2. Zugabein 380µl Lyse-Puffer +/- Lysozym
3. Zugabe von 200mg GlassBeads 600µm [Sigma-Aldrich Art, Nr, G8772-500G]
4. 5 min bei maximaler Leistung auf dem Vortex-Mischer mit Scheibenaufsatz für 1,5 ml Mikrozentrifugationsröhrchen mischen.
5. Zugabe von 600µl Puffer AL [QIAGEN; QIAamp DNA Micro Kit]
6. Zugabe von 20µl Proteinase K [QIAGEN; QIAamp DNA Micro Kit]
7. Mischen und Inkubation für 10min bei 56°C
8. Kurze Zentrifugation für 20 sec.
9. Transfer des gesamten Überstands in ein neues 1,5 ml Mikrozentrifugationsröhrchen
10. Zugabe von 600µl Ethanol
11. Gemisch auf QIAamp MinElute Spin Column geben (mehrmals)
12. *Bindung: 1 min bei 6000 x g zentrifugieren
13. *Waschschritte: 500µl Puffer AW1, 1 min bei 6000 x g ; 500µl Puffer AW2, 3min bei max. g-Zahl
14. *"Dry Spin für 1 min maximaler g-Zahl
15. *Zur Elution der Nukleinsäuren mit 80µl Puffer TE eluieren, 1 min bei 6000 x g
*) Schritte gemäß dem "QIAamp DNA Micro Handbook"

### Lyse Puffer

Lyse Puffer für Bakterien z.B. Corynebacterium glutamicum 1,2% Triton mit 20 mg/ml Lysozym
Zugabe von 10µl Reagent DX (Silicon-Antischaum Emulsion;
Wacker Chemie AG) auf 6ml Lysepuffer

Protokoll im Sinne der Erfindung
1. 200µl humanes Blut und 20 µl Corynebacterium glutamicum Überncichtkultur in 1 und in Lysekammer (siehe Figur 1) aliquotieren
2. Zugabein 380µl Lyse-Puffer +/- Lysozym
3. Zugabe von 200mg GlassBeads 600µm [Sigma-Aldrich Art. Nr. G8772-500G]
4. 10min Magnetrühren (zentrisch) bei größtmöglicher Umdrehungszahl, ohne dass es zur Verspritzung des Lysates kommt; Rührrichtung konstant
5. Zugabe von 600µl Puffer AL [QIAGEN; QIAamp DNA Micro Kit] Transfer mit aufgeschnittener Spitze in 1,5 ml Mikrozentrifugationsröhrchen
6. Zugabe von 20µl Proteinase K [QIAGEN; QIAamp DNA Micro Kit]
7. Mischen und Inkubation für 10min bei 56°C
8. Kurze Zentrifugation für 20 sec.
9. Transfer des gesamten Überstands in ein neues 1,5 ml Mikrozentrifugationsröhrchen
10. Zugabe von 600µl Ethanol
11. Gemisch auf QIAamp MinElute Spin Column geben (mehrmals)
12. *Bindung: 1 min bei 6000 x g zentrifugieren
13. *Waschschritte: 500µl Puffer AW1, 1 min bei 6000 x g ; 500µl Puffer AW2, 3min bei max, g-Zahl
14. *"Dry Spin für 1 min maximaler g-Zahl
15. *Zur Elution der Nukleinsäuren mit 80µl Puffer TE eluieren, 1 min bei 6000 x g

Folgende Versuche wurden durchgeführt.
K1; Referenzprotokoll mit Vortex-Mischer ohne Lysozym im Lysepuffer
K2: Referenzprotokoll mit Vortex-Mischer mit Lysozym im Lysepuffer
K3: Referenzprotokoll ohne Vortex-Mischen und ohne Lysozym im Lysepuffer
K4: Referenzprotoi<oll ohne Vortex-Mischen aber mit Lysozym im Lysepuffer
M1: Protokoll im Sinne der Erfindung ohne Lysozym im Lysepuffer
M2: Protokoll im Sinne der Erfindung mit Lysozym im Lysepuffer

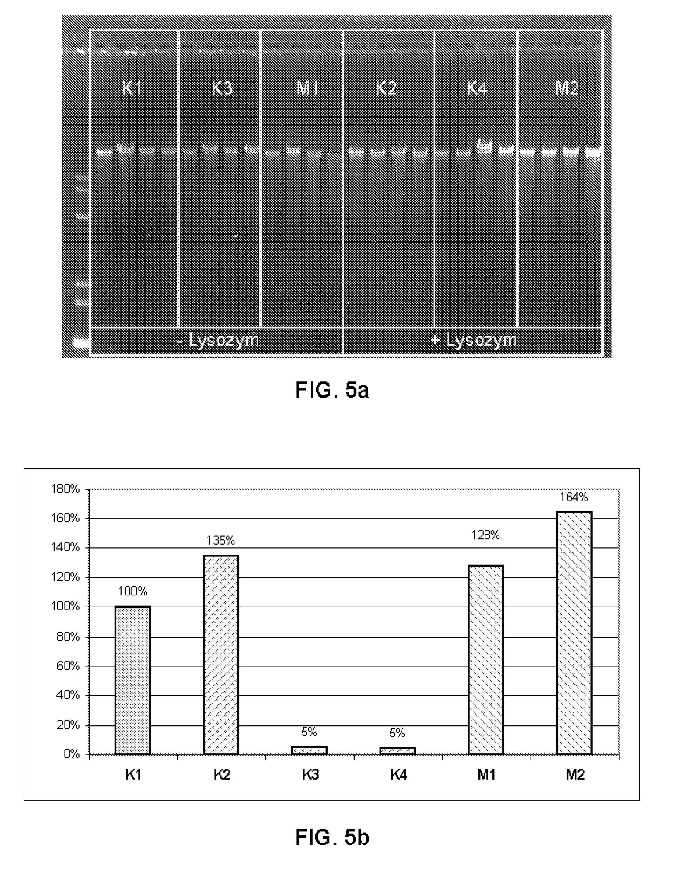

Fig. 5a verdeutlicht die qualitativ erzielten Ergebnisse einer Nukleinsäure-Präparation eines Gemisches aus humanen Blut und Corynebacterium glutamicum - Agarosegel der gesamten Nukleinsäure.

Fig. 5b zeigt das quantitativ erzielte Ergebnis der Nukleinsäure-Präparation eines Gemisches aus humanen Blut und Corynebacterium glutamicum - Ergebnisse der quantitativen PCR zum Nachweis genomischer Corynebacterium glutamicum DNA; relative Ausbeuten normalisiert auf das Refierenzprotokoll K1.

Wie aus den Figuren 5a und 5b ersichtlich, konnte das insgesamt beste Ergebnis mit Hilfe der Erfindung erzielt werden, nämlich gemäß Versuch M2, Durch den Einsatz von Lysozym kann die Lyseeffizienz bei Corynebacterium gluamicum deutlich verbessert werden.

## Patentansprüche

1. Verfahren für das Aufschließen von Zellen, indem Zellen zusammen mit Kügelchen oder Partikeln und einer Lösung, insbesondere einem Lysepuffer in ein Gefäß gegeben werden, **dadurch gekennzeichnet, dass** das Aufschließen durch Rühren beispielsweise mit einem Rührelement, insbesondere mit Hilfe eines Magnetrührers bewirkt wird,

2. Verfahren nach dem vorhergehenden Anspruch, bei dem Zellen von Bakterien oder Pilzen aufgeschlossen werden.

3. Verfahren nach einem der vorhergehenden Ansprüche, bei dem der Rührstab (3) des Magnetrührers stets schräg relativ zum Gefäßgrund (5) angeordnet ist.

4. Verfahren nach einem der vorhergehenden Ansprüche, bei dem ein Mischwerkzeug oder Rührelement (3) für das Rühren wenigstens 5 Minuten, vorzugsweise wenigstens 10 Minuten mit oder ohne Änderung der Drehrichtung gedreht wird.

5. Verfahren nach einem der vorhergehenden Ansprüche, bei dem im Anschluss an das Aufschließen die Pufferlösung mit den darin befindlichen aufgeschlossenen Zellen über eine Leitung (7) abgesaugt wird.

6. Verfahren nach einem der vorhergehenden Ansprüche, bei dem das Rührelement bezogen auf das Gefäß berührungsfrei angetrieben wird.

7. Verfahren nach einem der vorhergehenden Ansprüche, wobei die Lösung zusätzlich Enzyme für den Zellaufschluss enthält.

8. Vorrichtung für das Aufschließen von Zellen mit einem Gefäß (1), mit im Gefäß (1) befindlichen Kügelchen oder Partikel (4) und/ oder mit einer Leitung (7) für das Absaugen von Flüssigkeit aus dem Gefäß (1) heraus oder Zuführen von Flüssigkeit in das Gefäß (1) hinein, **dadurch gekennzeichnet, dass** ein Rührer, vorzugsweise ein Magnetrührer (3, 8, 9) für das Aufschließen von im Gefäß befindlichen Zellen vorhanden ist.

9. Vorrichtung nach dem vorhergehenden Anspruch, bei dem die Länge des Rührstabs (3) des Magnetrührers länger als der Durchmesser des Gefäßgrunds (5) ist.

10. Vorrichtung nach einem der beiden vorhergehenden Ansprüche mit einem Filter oder einer Fritte (5) für das Trennen der Kügelchen oder Partikel (4) von einer im Gefäß (1) befindlichen Flüssigkeit mit darin enthaltenen Zellen.

11. Vorrichtung nach dem vorhergehenden Anspruch, bei der die Fritte (5) den Grund des Gefäßes bildet und unterhalb der Fritte eine Leitung (7) für das Absaugen von Flüssigkeit aus dem Gefäß (1) heraus oder Zuführen von Flüssigkeit in das Gefäß (1) hinein angeordnet ist.

12. Vorrichtung nach einem der beiden vorhergehenden Ansprüche, bei der die Fritte (5) offene Poren mit einem Durchmesser von maximal 1 00 µm aufweist.

13. Vorrichtung nach einem der vorhergehenden Vorrichtungsansprüche, wobei der Durchmesser der Kügelchen oder Partikel (4) größer als 100 µm und/ oder kleiner als 1000 µm ist.

14. Vorrichtung nach einem der vorhergehenden Vorrichtungsansprüche, wobei das Gefäß (1) konisch ist und/ oder der Durchmesser des Gefäßes nach oben zunimmt.

15. Vorrichtung nach einem der vorhergehenden Vorrichtungsansprüche, wobei das Gefäß (1) einen Deckel (2) für das Verschließen des Gefäßes (1) umfasst.

16. Vorrichtung nach einem der vorhergehenden Vorrichtungsansprüche, wobei das Gefäß (1) auf einem Substrat (6) befestigt ist und eine Leitung (7) für das Absaugen von Flüssigkeit aus dem Gefäß (1) heraus oder Zuführen von Flüssigkeit in das Gefäß (1) im Substrat vorhanden ist.

17. Vorrichtung nach dem vorhergehenden Anspruch, bei dem das Substrat (6) unterhalb des Gefäßgrundes eine trichterförmige Ausnehmung aufweist.

18. Vorrichtung nach einem der vorhergehenden Vorrichtungsansprüche, wobei im Gefäß (1) Zellen von Bakterien oder Pilzen sind.
